# FASCICULE DE BREVET EUROPEEN

(11) **EP 0 286 501 B1**
(45) Date de publication et mention de la délivrance du brevet: **12.05.1993**
(21) Numéro de dépôt: 88400717.0
(22) Date de dépôt: 24.03.1988
(51) Int. Cl.: A61F 5/448

(54) **Dispositif de sécurité pour système d'appareillage des stomies**
Sicherheitsvorrichtung für einen Stoma-Apparat
Security device for a stoma apparatus

(30) Priorité: 10.04.1987 FR 8705146
(43) Date de publication de la demande: 12.10.1988
(73) Titulaire: B. BRAUN BIOTROL, 92100 Boulogne Billancourt (FR)
(72) Inventeur: Holtermann, Henri, F-64500 Saint-Jean-De-Luz (FR); Hamelin, Claude, F-64310 Ascain (FR)
(74) Mandataire: Epstein, Henri

(56) Documents cités:
- EP-A- 0 089 138
- EP-A- 0 163 979
- EP-A- 0 171 255
- EP-A- 0 255 310
- FR-A- 2 387 643
- US-A- 2 973 759

## Description

L'invention a pour objet un dispositif de sécurité pour système d'appareillage des stomies.

On connaît déjà, dans de nombreuses réalisations, des systèmes d'appareillage des stomies, c'est-à-dire des dispositifs permettant la fixation amovible au corps d'un utilisateur d'une poche de recueil de fluides et/ou de déchets corporels évacués par des patients ayant subi des interventions chirurgicales du tractus gastro-intestinal ou de l'appareil urinaire, comme des colostomies, des iléostomies, des urostomies, des urétérostomies, l'ensemble de ces interventions étant désigné ci-après sous le terme d'ostomie. De tels dispositifs sont décrits, par exemple, dans EP-A-0 089 138 qui forme le préambule de la revendication 1, EP-A-0 171 255 ou dans FR-A-2 387 643, relatifs l'un et l'autre à des dispositifs d'ostomie à poche collectrice sous forme d'un sac jetable, ou qui peut être vidé, et qui est solidarisé de manière amovible sur un dispositif de bague fixé au corps de l'utilisateur par un patin adhésif ou par une ceinture. La poche collectrice est mise en place par un encliquetage résultant d'une poussée ou pression qu'applique le patient autour de l'ouverture d'ostomie pour faire coopérer à emboîtement étanche les parties de forme conjuguée de la bague et d'une pièce solidaire de la poche. De tels dispositifs sont conçus pour que la fixation de la poche sur la bague par pression assure simultanément l'étanchéité du dispositif d'une part et, d'autre part, s'oppose à une séparation intempestive de la poche par rapport sa bague de montage. Pour l'obtention de résultats satisfaisants, les dispositifs connus exigent qu'une pression importante soit exercée par le patient lors de l'assemblage de la poche et de la bague. Etant donné, cependant, que la zone voisine d'ostomie est sensible, généralement douloureuse, l'application d'une forte pression ne fait qu'accroître le malaise de l'utilisateur, de sorte que les dispositifs connus du type mentionné ne sont pas entièrement satisfaisants.

On connaît également par EP-A1-0 255 310 publiée après la date de priorité un dispositif de sécurité pour système d'appareillage des stomies comportant deux éléments dont un est prévu pour être fixé autour d'une ouverture artificielle du corps de l'utilisateur à l'aide d'un moyen de fixation approprié tel, notamment, qu'un moyen adhésif sensible à la pression, une ceinture ou tout autre moyen analogue, et dont l'autre est solidaire d'une poche collectrice de recueil de fluides et/ou de déchets corporels destinée à être assemblée de manière amovible audit premier élément par emboîtement sous l'action d'une pression exercée lors du rapprochement des deux éléments susdits, l'élément qui est associé au patin formant un protecteur cutané de la zone péristomiale et autour du débouché d'une ou de sonde(s) ou analogue(s) qui en est (sont) solidaire(s) et comportant un premier embout tubulaire constitué d'une bague circulaire intérieure et d'une bague circulaire extérieure oui délimitent entre elles une gorge dans laquelle est propre à s'emboîter un second embout associé à la poche, ledit second embout étant propre à être immobilisé par rapport audit premier embout lorsque les deux embouts coopèrent à emboîtement par des moyens d'encliquetage élastiquement déformables prévus sur le premier embout, lequel comporte aussi, autour de lui, un organe rotatif pour le verrouillage desdits moyens d'encliquetage élastiquement déformables quand il est amené par rotation dans une position de blocage.

Compte tenu de cet état de la technique, la Demanderesse s'est fixé pour but de pourvoir à un dispositif de sécurité pour système d'appareillage des stomies qui soit d'utilisation moins contraignante et surtout moins désagréable pour le patient que celles des dispositifs connus, tout en assurant une parfaite sécurité de fixation, nonobstant l'utilisation d'une faible ou très faible pression pour la solidarisation de la poche et de la bague.

La présente invention a également pour but de permettre la fixation de ce dispositif, qui trouve application quel que soit le mode de fixation du patin portant la bague sur le corps de l'utilisateur, à l'aide d'un produit adhésif sensible à la pression ou à l'aide d'une ceinture.

C'est, aussi, un but de l'invention de pourvoir à un tel dispositif qui soit applicable non seulement dans le cas d'ostomie abdominale mais également pour des ostomies du système urinaire dans lesquelles on met parfois en oeuvre un système semi-permanent, c'est-à-dire un système dans lequel la poche ou sac de recueil des urines est muni d'un moyen d'évacuation de son contenu, de sorte que le dispositif n'est pas renouvelé à chaque à chaque miction mais est conservé par l'utilisateur pendant une durée plus longue que les dispositifs à usage unique.

C'est, alors, dans un tel cas d'utilisation, mais aussi pour un dispositif d'ostomie abdominale, un but de l'invention de fournir un dispositif qui permet de modifier légèrement, facilement et sans risque pour le patient, la position relative de la poche et de la bague, même lorsque ladite poche est partiellement remplie de fluides ou de déchets corporels.

La présente invention a pour objet un dispositif de sécurité pour système d'appareillage des stomies comportant deux éléments dont un est prévu pour être fixé autour d'une ouverture artificielle du corps de l'utilisateur à l'aide d'un moyen de fixation approprié tel, notamment, qu'un moyen adhésif sensible à la pression, une ceinture ou tout autre moyen analogue, et dont l'autre est solidaire d'une poche collectrice de recueil de fluides et/ou de déchets corporels destinée à être assemblée de manière amovible audit premier élément par emboîtement sous l'action d'une pression exercée lors du rapprochement des deux éléments susdits, l'élément qui est associé au patin formant un protecteur cutané de la zone péristomiale et autour du débouché d'une ou de sonde(s) ou analogue(s) qui en est (sont) solidaire(s) et comportant un premier embout tubulaire constitué d'une bague circulaire intérieure et d'une bague circulaire extérieure qui délimitent entre elles une gorge dans laquelle est propre à s'emboîter un second embout associé à la poche, ledit second embout étant propre à être immobilisé par rapport audit premier embout lorsque les deux embouts coopèrent à emboîtement par des moyens d'encliquetage élastiquement déformables prévus sur le premier embout, lequel comporte aussi, autour de lui, un organe rotatif pour le verrouillage desdits moyens d'encliquetage élastiquement déformables quand il est amené par rotation dans une position de blocage, caractérisé en ce que les moyens d'encliquetage sont logés dans des espaces de la bague circulaire extérieure du premier embout.

Selon un mode de réalisation particulièrement avantageux, lesdits moyens d'encliquetage sont constitués par des crochets d'attache à déformation élastique régulièrement disposés du point de vue angulaire sur la bague extérieure du premier embout, et présentant sur la face en regard de la bague intérieure, un bec qui immobilise le second embout quand celui-ci coopère avec le premier embout.

Selon une disposition avantageuse, le nombre de crochets d'attache est de 4, 6, 8 ou 12 en fonction du diamètre des embouts.

Selon un mode de réalisation préféré de l'invention, le second embout comporte en saillie sur sa face externe, une nervure annulaire à deux pans coupés qui se loge dans la gorge du premier embout, lorsque les deux embouts susdits sont emboîtés en effaçant les saillies des crochets d'attache.

Selon un autre mode de réalisation préféré de l'invention, l'organe rotatif pour le verrouillage comprend sur sa face interne des évidements régulièrement décalés du point de vue angulaire, dont le nombre correspond à celui des crochets d'attache disposés sur la bague extérieure du premier embout, et qui autorisent la déformation desdits crochets aussi bien pour l'emboîtement ou le désemboïtement desdits embouts.

Selon un mode de réalisation avantageux de l'invention, l'organe rotatif pour le verrouillage, moleté ou non sur sa face externe, présente un court levier facilitant la rotation partielle dudit organe autour du premier embout.

Selon une autre caractéristique de l'invention, on prévoit sur la face externe de la bague extérieure du premier embout, un plot de blocage de l'organe rotatif, logé, en condition opératoire, dans une rainure d'ouverture angulaire prédéterminée prévue sur la face interne de l'organe rotatif pour le verrouillage, ce qui définit la course de l'organe rotatif de verrouillage.

L'invention a, de plus, pour objet un protecteur cutané propre à entrer dans la constitution d'un tel dispositif comprenant sur un patin propre à être fixé au corps de l'utilisateur à l'aide d'un moyen de fixation approprié tel, notamment, qu'un moyen sensible à la pression, une ceinture ou tout autre moyen analogue, un embout tubulaire en saillie sur la face dudit patin opposée à celle destinée à venir au contact du corps de l'utilisateur, ledit embout comportant une bague intérieure circulaire et une bague extérieure circulaire co-axiales délimitant entre elles une gorge dans laquelle est propre à s'emboîter l'embout associé à une poche et, autour du premier embout, un organe rotatif de verrouillage qui bloque des moyens d'encliquetage élastiquement déformables prévus sur le premier embout lorsqu'il est amené par rotation dans une position de blocage, caractérisé en ce que la bague circulaire présente des espaces dans lesquels sont logés les moyens d'encliquetage et en ce que l'organe de verrouillage comprend sur sa face interne des évidements dont la position et le nombre correspondent à ceux des moyens d'encliquetage.

Outre les dispositions qui précèdent, l'invention comprend encore d'autres dispositions, qui ressortiront de la description qui va suivre.

L'invention sera mieux comprise par la description qui suit, faite à titre d'exemple et en référence aux dessins annexés.

Il doit être bien entendu, toutefois, que ces dessins et les parties descriptives correspondantes, sont donnés uniquement à titre d'illustration de l'objet de l'invention, dont ils ne constituent en aucune manière une limitation.
- la figure 1 est une vue schématique, en perspective, des deux éléments constitutifs d'un dispositif selon l'invention,
- la figure 2 est une vue en plan de dessus, d'un embout de dispositif selon l'invention,
- la figure 3 est une vue en plan de dessus de l'organe rotatif pour le verrouillage selon l'invention,
- la figure 4 est une vue en plan de dessus d'un embout de dispositif selon l'invention sur lequel est disposé l'organe rotatif pour le verrouillage,
- la figure 5 est une section d'un crochet d'attache d'un embout de dispositif selon l'invention,
- la figure 6 est une section de l'autre embout de dispositif selon l'invention,
- la figure 7 est une demi-vue en plan lorsque les deux embouts de dispositif selon l'invention sont dans la condition d'emboîtement,
- la figure 8 est une section suivant la ligne 8-8 de la figure 7, à plus grande échelle,
- la figure 9 est une section suivant la ligne 9-9 de la figure 7, à plus grande échelle,
- la figure 10 est une section suivant la ligne 10-10 de la figure 7, à plus grande échelle,
- la figure 11 est une vue en coupe d'un dispositif selon l'invention lorsque les deux embouts sont dans la condition d'emboîtement,
- la figure 12 est une vue partielle, à plus grande échelle, analogue à la figure 2, mais pour une variante,
- la figure 13 est une vue en coupe selon la ligne 13-13 de la figure 12.

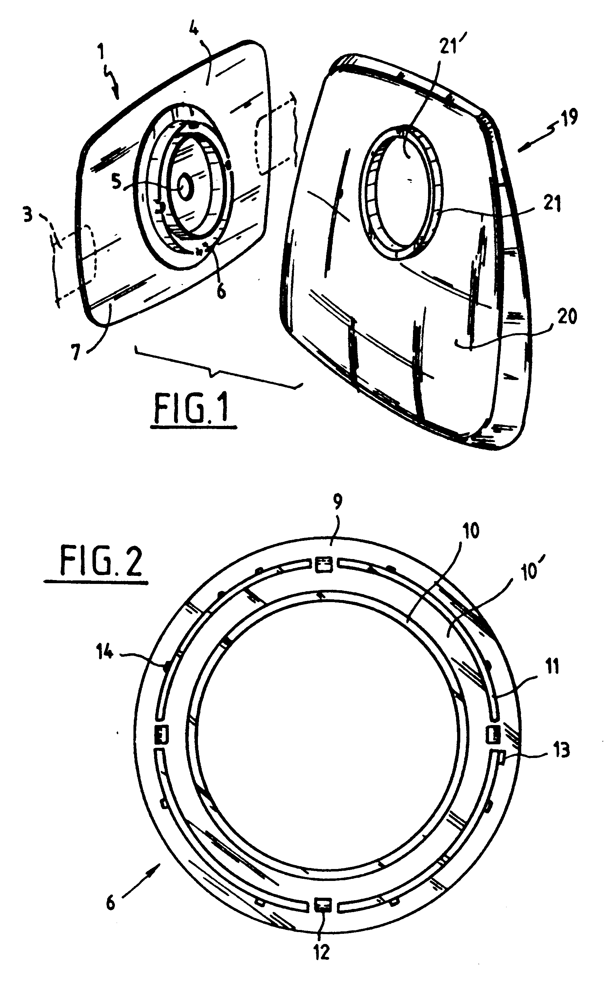

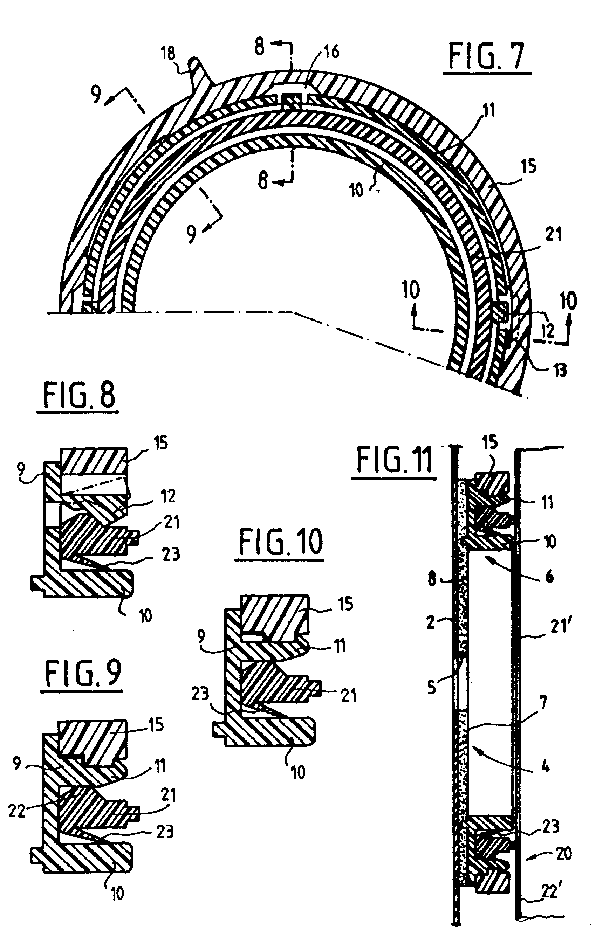

Les figures 1 à 10 sont relatives à un dispositif de sécurité pour système d'appareillage des stomies selon l'invention. Celui-ci comprend un élément 1, prévu pour être fixé autour d'une ouverture artificielle du corps de l'utilisateur à l'aide d'un moyen de fixation approprié tel qu'un moyen adhésif sensible à la pression, en soi connu, et qui est protégé, aussi longtemps que le dispositif n'est pas mis en oeuvre, par une pellicule 2 qui peut être aisément retirée par pelage (figure 11), ou une ceinture 3 ou tout autre moyen analogue.

L'élément 1 comprend essentiellement un patin 4 en un matériau souple, qui peut être de forme carrée, rectangulaire, ronde ou polygonale par exemple, destiné à former une protection cutanée de la zone péristomiale autour de laquelle il est placé et maintenu. Cet élément 1 présente une ouverture 5 concentrique ou sensiblement concentrique à la stomie, et un embout 6 en saillie sur la face 7 du patin 4 opposée à celle portant le matériau adhésif 8, figure 11, ou lorsqu'un tel matériau n'est pas présent, opposée à la face en contact avec la peau de l'utilisateur.

L'embout 6 est rendu solidaire du patin 4 par soudure, par exemple du type thermique ou haute fréquence ou encore par collage par l'intermédiaire de films compatibles avec le matériau constitutif de l'embout 6.

Le patin 4 peut être constitué à partir d'une masse adhésive hydrophile d'une épaisseur comprise entre 0,5 et 3 mm, ou à partir d'une masse adhésive acrylique très mince dont l'épaisseur est comprise entre quelques microns et 200 microns environ ou encore par combinaison de ces deux éléments, c'est-à-dire, en prévoyant autour de la stomie une gomme adhésive hydrophile et, à la périphérie de celle-ci, une masse adhésive acrylique mince.

L'embout 6 peut être réalisé en polyéthylène, haute ou basse densité, ou en copolymère d'éthylène et d'acétate de vinyle (EVA) ou en polychlorure de vinyle (PVC) ou en un polyamide, par moulage par exemple, et les films compatibles pour la fixation de l'embout sur le protecteur cutané sont alors avantageusement des films de polyéthelène, de PVC, de polyamide, ou des films barrière complexes ou des non-tissés à base de polyester, de polypropylène et/ou de polyéthylène.

L'embout 6 comprend une embase 9 circulaire, qui porte une bague circulaire intérieure 10 et une bague circulaire extérieure 11 coaxiale à la première et régulièrement découpée par des espaces dans lesquels sont logés des crochets d'attache 12, lesdites bagues coaxiales délimitant entre elles une gorge 10ʹ dans laquelle vient s'emboîter le second embout 21 associé à la poche 20. Les crochets d'attache 12 présentent sur la face en regard de la bague intérieure 10, un bec 12ʹ qui immobilise le second embout 21 solidaire de la poche 20 de recueil des fluides et/ou des déchets corporels quand ledit embout 21 coopère avec l'embout 6 dans la condition d'embotement.

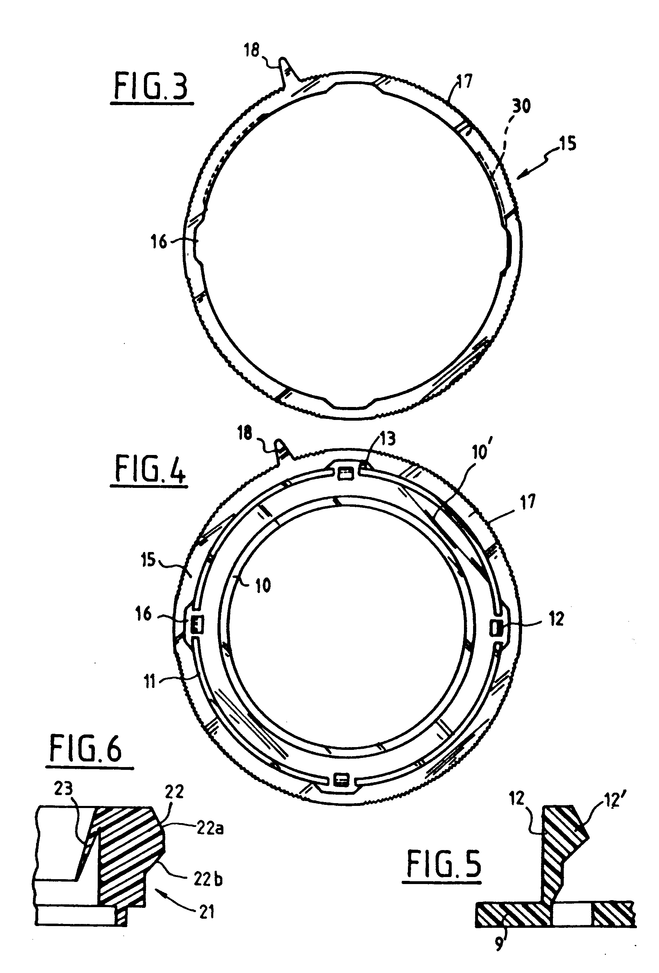

La bague circulaire extérieure 11 présente sur le pourtour de sa face externe de légers renflements 14, qui empêchent l'extraction d'un organe rotatif de verrouillage 15 lorsque ce dernier est disposé autour de la bague 11, celle-ci comportant également sur sa face externe un plot 13 de limitation de l'envergure de rotation de l'organe rotatif 15, logé en condition opératoire dans une rainure 30 d'ouverture angulaire prédéterminée, par exemple 45°, prévue sur la face interne de l'organe rotatif 15, figure 3.

Ledit organe est ou non moleté sur sa face externe 13 et présente un court levier 18 qui facilite la rotation partielle dudit organe autour de la bague circulaire extérieure 11. L'organe rotatif 15 présente également, sur sa face interne, des évidements 16 régulièrement décalés du point de vue angulaire, dont le nombre correspond à celui des crochets d'attache 12, lesdits évidements autorisant la déformation élastique des crochets 12 lorsqu'on emboîte les deux embouts 6 et 21 et/ou lorsqu'on les extraie l'un de l'autre.

Le dispositif de sécurité pour système d'appareillage des stomies selon l'invention comprend également un élément 19 propre à coopérer, de manière amovible, avec l'élément 1. Cet élément est constitué essentiellement de la poche 20 de recueil de fluides et/ou déchets corporels propres à être évacués au travers de l'embout 6 et qui comprend une paroi 22ʹ, (figure 11), percée d'un trou 21ʹ par lequel les fluides ou déchets corporels pénètrent à l'intérieur de la poche, laquelle peut être du type à jeter, ou à vider, en fonction des desiderata de la pratique. Elle peut être réalisée en un film de polyéthylène, ou de PVC, ou de polyamide (comme celui connu sous la marque déposée RILSAN) ou en un film barrière complexe du type polyéthylène/EVA/polychlorure de vinylidène/EVA/polyéthylène, comme ceux connus sous la marque déposée SARANEX de la Société DOW CHEMICAL, ou un film complexe du type EVA/copolymère polychlorure de vinylidène-EVA, comme ceux connus sous la marque CRYOVAC de la Société GRACE et sous la marque déposée SARANEX de la Société DOW CHEMICAL, ou encore en film complexe du type EVA/EVOH/EVA, ou en caoutchouc ou analogue.

Conformément à l'invention, à la poche 20 est associé l'embout 21, avantageusement réalisé en la même matière plastique que l'embout 6, par exemple en polyéthylène haute densité, et solidarisé de manière étanche avec la poche 20, sur laquelle il est soudé ou collé, comme indiqué ci-dessus, de manière que son axe soit coaxial à celui du trou 21ʹ.

Ledit embout 21 comporte en saillie sur sa face externe une nervure annulaire 22 à deux pans coupés 22a, 22b qui se loge dans la gorge 10ʹ, en effaçant élastiquement les becs 12ʹ des crochets 12 du premier embout 6 lorsque les deux embouts 6 et 21 sont emboîtés. L'embout 21 comporte également en saillie sur sa face interne une lévre annulaire plastique déformable 23 qui, de façon en soi connue, assure l'étanchéité du dispositif à l'encontre des fluides et/ou déchets recueillis.

Le fait que l'embout 21 est de forme et de dimension conjuguées par rapport à l'embout 6 contribue à cette étanchéité.

Pour la mise en place du dispositif, on réalise l'emboîtement de l'embout 21 dans l'embout 6 en s'assurant au préalable que l'organe rotatif pour le verrouillage 15 est disposé de manière que les évidements 26 se présentent en face des crochets d'attache 12. Lors de l'emboîtement, les crochets sont déformés élastiquement (position en trait mixte de la figure 8) et pénètrent dans les évidements 16, quand l'embout 21 est positionné au fond de la gorge 10ʹ, de l'embout 6, lesdits crochets reprennent leur position initiale (position en trait plein de la figure 8) en assurant le maintien de l'embout 21 dans ladite gorge 10ʹ par les becs 12ʹ qui se positionnent alors contre l'un des pans coupés 22b de la nervure annulaire 22 dudit embout 21. On procède alors à la rotation partielle de l'organe rotatif 15 pour le verrouillage de la poche 20 par rapport au patin 4, cette rotation étant limitée en fin de course par butée du plot de blocage 13 sur l'un des bords de la rainure 30 d'ouverture angulaire prédéterminée prévue sur la face interne de l'organe rotatif pour le verrouillage 15. Les deux embouts 6 et 21 étant alors emboîtés et verrouillés, une rotation de l'élément 19 par rapport à l'élément 1 est possible et le patient peut en tirer parti, pour modifier légèrement la position de la poche 20, même lorsque celle-ci est partiellement remplie, sans la désolidariser du patin 4.

C'est un processus inverse qui est mis en oeuvre pour séparer la poche 20 du patin 4 après rotation de l'organe 15 pour deverrouiller les crochets 12.

Ainsi que cela ressort de ce qui précède, l'invention ne se limite nullement à ceux de ses modes de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

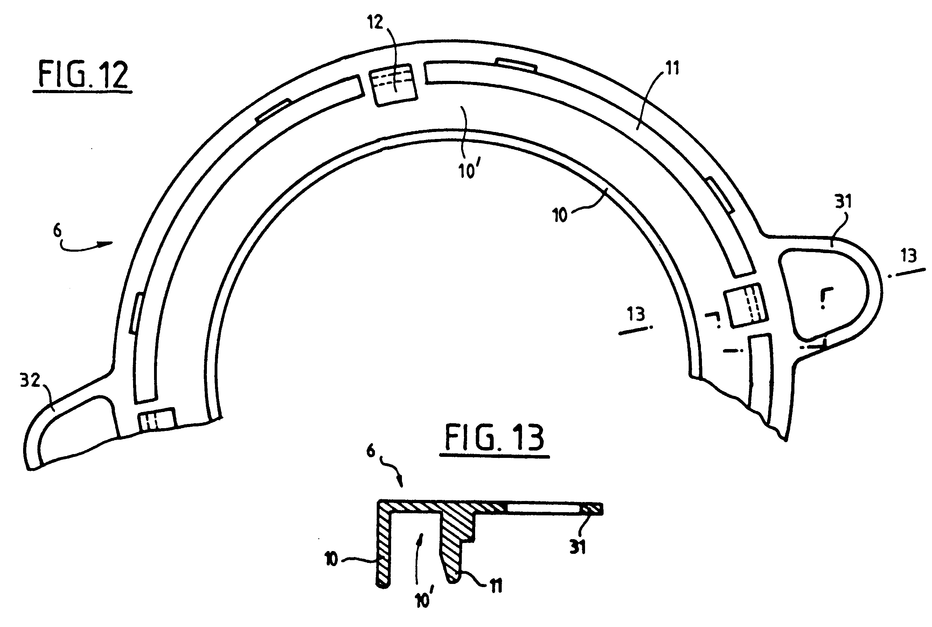

Ainsi, et comme montré sur les figures 12 et 13, l'embase 9 de l'embout 6 peut aussi être munie en des zones diamétralement opposées d'oreilles 31 et 32 d'attache d'une ceinture non représentée. Une telle réalisation qui comprend également un patin 4 à adhésif sensible à la pression est d'un intérêt particulier pour les patients présentant une stomie invaginée.

Lorsque le dispositif de l'Invention est utilisé pour des urostomies ou des urétérostomies au moins un ou plusieurs cathéters, des sondes ou analogues, peuvent être fixés au patin, comme connu en soi, et sans que cela entraîne de modifications de structure des autres parties du dispositif selon l'invention.

## Revendications

1. Dispositif de sécurité pour système d'appareillage des stomies comportant deux éléments (1, 19) dont un est prévu pour être fixé autour d'une ouverture artificielle du corps de l'utilisateur à l'aide d'un moyen de fixation approprié tel, notamment, qu'un moyen adhésif sensible à la pression, une ceinture ou tout autre moyen analogue, et dont l'autre est solidaire d'une poche collectrice de recueil de fluides et/ou de déchets corporels (20), destinée à être assemblée de manière amovible audit premier élément par emboîtement sous l'action d'une pression exercée lors du rapprochement des deux éléments susdits, l'élément (1) qui est associé au patin (4) formant un protecteur cutané de la zone péristomiale et autour du débouché d'une ou de sonde(s) ou analogue(s) qui en est (sont) solidaire(s) et comportant un premier embout (6) tubulaire constitué d'une bague circulaire intérieure (10) et d'une bague circulaire extérieure (11) qui délimitent entre elles une gorge (10') dans laquelle est propre à s'emboîter un second embout (21) associé à la poche (20), ledit second embout (21) étant propre à être immobilisé par rapport audit premier embout (6) lorsque les deux embouts (6, 21) coopèrent à emboîtement par des moyens d'encliquetage élastiquement déformables (12) prévus sur le premier embout (6), caractérisé en ce qu'il comporte autour du premier embout (6) un organe rotatif (15) pour le verrouillage desdits moyens d'encliquetage élastiquement déformables (12) quand il est amené par rotation dans une position de blocage, les moyens d'encliquetage (12) sont logés dans des espaces de la bague circulaire extérieure (11) du premier embout (6).

2. Dispositif selon la revendication 1, caractérisé en ce que lesdits moyens d'encliquetage sont constitués par des crochets d'attache (12) à déformation élastique régulièrement disposés du point de vue angulaire sur la bague extérieure (11) du premier embout et présentant, sur la face en regard de la bague intérieure (10), un bec (12') qui immobilise le second embout (21) quand celui-ci coopère avec le premier embout (6).

3. Dispositif selon la revendication 2, caractérisé en ce que le nombre de crochets d'attache (12) est de 4, 6, 8 ou 12 en fonction du diamètre des embouts (6, 21).

4. Dispositif selon la revendication 1, caractérisé en ce que le second embout (21) comporte en saillie sur sa face externe, une nervure annulaire (22) à deux pans coupés (22a, 22b), qui se loge dans la gorge (10') du premier embout (6), lorsque les deux embouts (6, 21) susdits sont emboîtés en effaçant les saillies (12') des crochets d'attache (12).

5. Dispositif selon l'une quelconque des revendications 2 à 4, caractérisé en ce que l'organe rotatif pour le verrouillage (15) comprend sur sa face interne des évidements (16) régulièrement décalés du point de vue angulaire, dont le nombre correspond à celui des crochets d'attache (12) disposés sur la bague extérieure du premier embout et qui autorisent la déformation desdits crochets (12) pour l'emboîtement ou le désemboîtement desdits embouts (6, 21).

6. Dispositif selon la revendication 5, caractérisé en ce que l'organe rotatif pour le verrouillage (15) est ou non moleté sur sa face externe (17) et présente un court levier (18) facilitant la rotation partielle dudit organe autour du premier embout (6).

7. Dispositif selon l'une quelconque des revendications précédentes, caractérisé en ce que l'on prévoit sur la face externe de la bague extérieure (11) du premier embout (6), un plot de blocage (13) de l'organe rotatif (15), logé en condition opératoire dans une rainure (30) d'ouverture angulaire prédéterminée prévue sur la face interne de l'organe rotatif (15) pour le verrouillage.

8. Dispositif selon l'une quelconque des revendications précédentes, caractérisé en ce que l'embout (6) solidaire du patin (4) est formé sur une embase (9) et en ce que cette dernière présente des oreilles (31, 32) ou analogues d'attache d'une ceinture de fixation (3).

9. Protecteur cutané propre à entrer dans la constitution d'un dispositif d'ostomie selon l'une quelconque des revendications 1 à 8, comprenant sur un patin (4) propre à être fixé au corps de l'utilisateur à l'aide d'un moyen de fixation approprié tel, notamment, qu'un moyen sensible à la pression, une ceinture (3) ou tout autre moyen analogue, un embout tubulaire (6) en saillie sur la face dudit patin opposée à celle destinée à venir au contact du corps de l'utilisateur, ledit embout comportant une bague intérieure circulaire (10) et une bague extérieure circulaire (11) coaxiales délimitant entre elles une gorge (10') dans laquelle est propre à s'emboîter l'embout (21) associé à une poche (20) et, autour du premier embout (6), un organe rotatif de verrouillage (15) qui bloque des moyens d'encliquetage (12) élastiquement déformables prévus sur le premier embout (6) lorsqu'il est amené par rotation dans une position de blocage, caractérisé en ce que la bague circulaire (11) présente des espaces dans lesquels sont logés les moyens d'encliquetage (12) et en ce que l'organe de verrouillage (15) comprend sur sa face interne (16) des évidements dont la position et le nombre correspondent à ceux des moyens d'encliquetage (12).

## Claims

1. A safety device for an ostomy apparatus system comprising two elements (1,19) one of which is provided for fixing around an artificial opening in the body of the user by appropriate fixing means, in particular by pressure sensitive adhesive, a belt, or any other analogous means, and the other of which is fixed to a collector bag for collecting body fluids and/or wastes (20), and intended to be removeably assembled to said first element by interfitting under the effect of pressure exerted when the two above-mentioned elements are moved towards each other; the element (1) which is associated with the plate (4) constituting a skin protector for the peristomial zone and disposed around the opening of one or more sondes or the like fixed thereto, and comprising a first tubular end piece (6), constituted by an inner circular ring (10) and an outer circular ring (11), which between them delimit a groove (10') in which the second end piece (21) associated with the bag (20) is suitable for being engaged, the said second end piece (21) being suitable for being fixed relative to the said first end piece (6) when the two end pieces (6,21) co-operate by engaging of resiliently deformable snap-fastening means (12) provided on the first end piece (6), characterized in that it comprises a rotary locking member (15) around the first end piece (6) for locking together the said resiliently deformable snap-fastening means (12) when it is rotated into a locking position, the snap-fastening means (12) are received in spaces in the outer circular ring (11) of the first end piece (6).

2. A device according to claim 1, characterized in that said snap-fastening means are constituted by resiliently deformable fastening hooks (12) regularly disposed angularly on the outer ring (11) of the first end piece, and having respective catches (12') on their faces facing the inner ring (10), said catches preventing the second end piece (21) from moving when it co-operates with the first end piece (6).

3. A device according to claim 2, characterized in that the number of fastening hooks (12) is 4, 6, 8, or 12 as a function of the diameter of the end pieces (6,21).

4. A device according to claim 1, characterized in that the second end piece (21) includes an annular rib (22) with two sloping surfaces (22a, 22b) projecting from its outside face, said ring being received in the groove (10') of the first end piece (6), when the two above-mentioned end pieces (6,21) are engaged by pushing back the projections (12') of the fastening hooks (12).

5. A device according to any one of claims 2 to 4, characterized in that the rotary locking member (15) includes regularly angularly spaced-apart hollows (16) on its inside face, with the number of hollows being the same as the number of fastening hooks (12) disposed on the outer ring of the first end piece and enabling said hooks (12) to be deformed for the purpose of engaging or disengaging said end pieces (6,21).

6. A device according to claim 5, characterized in that the rotary locking member (15) is optionally knurled on its outer face (17) and has a short lever (18) facilitating partial rotation of said member around the first end piece (6).

7. A device according to any preceding claim, characterized in that a locking tab (13) for the rotary member (15) is provided on the outside face of the outer ring (11) of the first end piece (6), said tab being received in the operating condition in a groove (30) of predetermined annular extent provided in the inside face of the rotary locking member.

8. A device according to any preceding claim, characterized in that the end piece (6) fixed to the plate (4) is formed on a base (9), and in that the base has lugs (31, 32) or the like for connection to a fixing belt.

9. A skin protector suitable for forming a part of an ostomy device according to any one of claims 1 to 8, characterized in that it comprises a tubular end piece (6) on a plate (4) suitable for being fixed to the body of the user by any appropriate fixing means such as a belt (3), pressure-sensitive means, or any other analogous means, the tubular end piece (6) projecting from the face of said plate opposite to its face for coming into contact with the body of the user, the said end piece comprising a circular inner ring (10) and a circular outer ring (11) which are coaxial, delimiting between them a groove (10') in which the end piece (21) associated with the bag (20) is suitable to be engaged and, around the first end piece (6), a rotary locking member (15) which locks the resiliently deformable snap-fastening means (12) provided on the first end piece (6) when it is rotated into a locked position, characterized in that the circular ring (11) includes spaces in which the snap-fastening means (12) are received and in that the locking member (15) includes hollows on its inside face (16) whose positions and number correspond to those of the snap-fastening means (12).

## Patentansprüche

1. Sicherheitsvorrichtung für ein System betreffend einen künstlichen Körperausgang bestehend aus zwei Elementen (1, 19), wovon eines dazu vorgesehen ist, um rund um einen künstlichen Ausgang des Körpers des Benützers mit Hilfe eines geeigneten Befestigungsmittels, wie insbesondere eines druckempfindlichen Haftmittels, eines Gürtels oder jedes anderen ähnlichen Mittels befestigt zu werden, und das andere fest mit einem Sammelbeutel zum Sammeln von Flüssigkeiten und/oder Körperausscheidungen (20) verbunden ist, der durch Einrasten unter Ausübung eines Druckes bei Annäherung beider Elemente lösbar an das erste Element anschließbar ist, wobei das Element (1), welches eine Unterlage (4) zugeordnet ist, die einen Hautschutz der peristomialen Zone und rund den Ausgang einer oder mehrerer Sonden oder ähnlichem bildet, die mit ihr fest verbunden sind, und ein erstes röhrenförmiges Ansatzstück (6) aufweisen, welches aus einem kreisförmigen inneren Ring (10) und einem kreisförmigen äußeren Ring (11) besteht, welche zwischen ihnen einen Hals (10') begrenzen, in welchem ein Zweites Ansatzstück (21) angeschlossen werden kann, das dem Beutel (20) zugeordnet ist, wobei das Zweite Ansatzstück (21) dazu geeignet ist, bezüglich des ersten Ansatzstücks (6) fixiert zu werden, wenn die Zwei miteinander verbundenen Ansatzstücke (6, 21) mit einer elastisch deformierbaren Einklinkvorrichtung (12) zusammenwirken, welche an dem ersten Ansatzstück (6) vorgesehen ist, dadurch gekennzeichnet, daß sie rund um das röhrenförmige Ansatzstück (6) einen drehbaren Teil (15) aufweist, um an der elastisch deformierbaren Einklinkvorrichtung (12) einzurasten, wenn sie durch Drehen in die Verriegelungsstellung gebracht wird, wobei die Einklinkvorrichtung (12) in den Zwischenräumen des kreisförmigen äußeren Ringes (11) des ersten Ansatzstücks (6) untergebracht ist.

2. Vorrichtung nach Anspruch 1, dadurch gekennzeichnet, daß die Einklinkvorrichtungen aus elastisch verformbaren Befestigungshaken (12) bestehen, welche in regelmäßigen Winkelabständen auf dem äußeren Ring (11) des ersten Ansatzstückes angeordnet sind, und welche auf der Seite, die dem inneren Ring (10) zugewandt ist, einen Vorsprung (12') aufweisen, der das zweite Ansatzstück (21) beim Zusammenfügen mit dem ersten Ansatzstück (6) fixiert.

3. Vorrichtung nach Anspruch 2, dadurch gekennzeichnet, daß die Anzahl der Befestigungshaken (12) in Abhängigkeit des Durchmessers der Ansatzstücke (6, 21) 4, 6, 8 oder 12 beträgt.

4. Vorrichtung nach Anspruch 1, dadurch gekennzeichnet, daß das zweite Ansatzstück (21) an seiner Außenseite eine vorspringende ringförmige Rippe (22) mit zwei schrägen Flächen (22a, 22b) aufweist, welche sich in die Rille (10') des ersten Ansatzstückes (6) einpaßt, wenn die beiden oben genannten Ansatzstücke (6, 21) eingerastet werden, indem die Vorsprünge (12') der Befestigungshaken (12) zurückweichen.

5. Vorrichtung nach einem der Ansprüche 2 bis 4, dadurch gekennzeichnet, daß der drehbare Teil zur Verriegelung (15) an seiner inneren Fläche Auskehlungen (16) aufweist, welche in gleichen Winkelabstanden regelmäßig versetzt sind und deren Anzahl derjenigen der Befestigungshaken (12) entspricht, welche an dem äußeren Ring des ersten Ansatzstückes angeordnet sind, und welche die Verformung dieser Haken (12) zum Einrasten und Ausrasten der Ahsatzstücke (6, 21) gewährleisten.

6. Vorrichtung nach Anspruch 5, dadurch gekennzeichnet, daß der drehbare Teil zur Verriegelung (15) auf seiner äußeren Berandung (17) gerändelt ist oder nicht und einen kurzen Hebel (18) aufweist, welcher eine teilweise Verdrehung dieses Teils um das erste Ansatzstück (6) erleichtert.

7. Vorrichtung nach einem der vorgehenden Ansprüche, dadurch gekennzeichnet, daß auf der äußeren Fläche des äußeren Ringes (11) des ersten Ansatzstückes (6) ein Blockiersteckstück (13) für den drehbaren Teil (15) vorgesehen ist, welches sich im Betriebszustand in einem Schlitz (30) mit vorgegebener Winkelöffnung befindet, der auf der inneren Fläche des drehbaren Verriegelungsteiles (15) vorgesehen ist.

8. Vorrichtung nach einem der vorgehenden Ansprüche, dadurch gekennzeichnet, daß das Ansatzstück (6), das mit der Unterlage (4) fest verbunden ist, auf einem Unterteil (9) ausgebildet ist und daß letzterer Befestigungsschlaufen (31, 32) oder ähnliches für einen Befestigungsgürtel (3) aufweist.

9. Hautschutz, der für den Einbau in eine Vorrichtung für einen künstlichen Körperausgang nach einem der Ansprüche 1 bis 8 geeignet ist, der auf einer Unterlage (4), die mittels eines geeigneten Befestigungsmittels, wie insbesondere eines druckempfindlichen Mittels, eines Gürtels (3) oder jedes anderen ähnlichen Mittels an dem Körper des Benützers befestigbar ist, ein röhrenförmiges Ansatzstück (6) aufweist, das auf der Fläche der Unterlagen vorspringt, welche jener gegenüberliegt, die mit dem Körper des Benützers in Kontakt kommt, und welches einen inneren kreisförmigen Ring (10) und einen koaxialen äußeren kreisförmigen Ring (11) aufweist, die dazwischen koaxial einen Hals (10') begrenzen, in welchem das Ansatzstück (21), welches einem Beutel (20) zugeordnet ist, und rund um das erste Ansatzstück (6) ein drehbarer Teil (15) zur Verriegelung angeschlossen werden kann, der die elastisch deformierbare, an dem ersten Ansatzstück vorgesehene Einklinkvorrichtung (12) blockiert, nachdem er durch Drehung in eine Verriegelungsstellung gebracht wird, dadurch gekennzeichnet, daß der kreisförmige Ring (11) Unterbrechungen aufweist, in welchen sich die Einklinkvorrichtung (12) befindet, und daß der Verriegelungsteil (15) an seiner Innenfläche (16) Vorsprünge aufweist, deren Position und Zahl jenen der Einklinkvorrichtung (12) entspricht.
